Europäisches Patentamt

European Patent Office

Office européen des brevets

(19)

(11) EP 0 692 981 B1

(12) EUROPÄISCHE PATENTSCHRIFT

(45) Veröffentlichungstag und Bekanntmachung des Hinweises auf die Patenterteilung:
21.04.1999 Patentblatt 1999/16

(21) Anmeldenummer: 94912442.4

(22) Anmeldetag: 29.03.1994

(51) Int. Cl.⁶: A61K 51/00

(86) Internationale Anmeldenummer:
PCT/DE94/00372

(87) Internationale Veröffentlichungsnummer:
WO 94/22493 (13.10.1994 Gazette 1994/23)

(54) **CHELATBILDNER VOM TYP XN 1?S 1?X' FÜR RADIOAKTIVE ISOTOPE, DEREN METALLKOMPLEXE UND IHRE VERWENDUNG IN DIAGNOSTIK UND THERAPIE**

CHELATORS OF TYPE XN 1?S 1?X' FOR RADIOACTIVE ISOTOPES, THEIR METAL COMPLEXES AND THEIR DIAGNOSTIC AND THERAPEUTICAL USES

AGENTS CHELATEURS DE TYPE XN 1?S 1?X' D'ISOTOPES RADIOACTIFS, LEUR COMPLEXES METALLIQUES ET LEURS UTILISATIONS DIAGNOSTIQUES ET THERAPEUTIQUES

(84) Benannte Vertragsstaaten:
AT BE CH DE DK ES FR GB GR IE IT LI LU MC NL PT SE

(30) Priorität: 31.03.1993 DE 4310999

(43) Veröffentlichungstag der Anmeldung:
24.01.1996 Patentblatt 1996/04

(73) Patentinhaber:
INSTITUT FÜR DIAGNOSTIKFORSCHUNG GmbH
AN DER FREIEN UNIVERSITÄT BERLIN
14050 Berlin (DE)

(72) Erfinder:
• HILGER, Christoph-Stephan
  D-13353 Berlin (DE)
• DINKELBORG, Ludger
  D-13585 Berlin (DE)
• KRAMP, Wolfgang
  D-13503 Berlin (DE)
• SCHIER, Hans-Martin
  15344 Strausberg (DE)

(74) Vertreter: Mager, Knut
Rechtsanwalt,
Müllerstrasse 178
13353 Berlin (DE)

(56) Entgegenhaltungen:
EP-A- 0 299 795

• CHEMICAL ABSTRACTS, vol. 119, no. 13, 27. September 1993, Columbus, Ohio, US; abstract no. 139794, & JP,A,5 070 484 (HITACHI CHEMICAL CO LTD)
• TETRAHEDRON, Bd.38, Nr.14, 1982, OXFORD GB Seiten 2061 - 2067 R. MARCHELLI ET AL. 'CHIRAL AMINOACID CONTAING LIGANDS-II. COMPLEXATION OF ALKALINE EARTH CATIONS'

**Beschreibung**

Die Erfindung betrifft neue bifunktionelle chalkogenatom-unterbrochene Chelatbildner, diese Verbindungen enthaltende pharmazeutische Mittel, ihre Verwendung in der Radiodiagnostik und Radiotherapie sowie Verfahren zur Herstellung dieser Verbindungen und Mittel.

Die Anwendungen von Komplexbildnern für radioaktive Isotope bzw. ihre Komplexe mit radioaktiven Metallen in der Radiodiagnostik und Radiotherapie ist seit langem bekannt. Für die Radiodiagnostik wird am häufigsten das Radionuklid Technetium-99m verwendet, das auf Grund seiner günstigen physikalischen Eigenschaften (keine Korpuskularstrahlung, geringe Halbwertzeit von 6.02 h, gute Detektierbarkeit durch 140 keV $\gamma$-Strahlung) und geringe biologische Halbwertzeit und einfache Verfügbarkeit besonders gut für eine in vivo Anwendung geeignet ist. Zur Bildung von Technetium-99m-Komplexen wird Pertechnetat zunächst aus einem Nuklidgenerator gewonnen und durch Verwendung geeigneter Reduktionsmittel (z. B. $SnCl_2$, $S_2O_4^{2-}$ etc.) in eine niedrigere Oxidationsstufe überführt, die anschließend durch einen geeigneten Chelator stabilisiert wird. Da Technetium in einer Reihe von Oxidationsstufen (+ 7 bis - 1) vorliegen kann, die die pharmakologischen Eigenschaften durch Veränderungen der Ladung eines Komplexes stark verändern können, ist es notwendig, Chelatoren bzw. Komplexliganden für Technetium-99m bereitzustellen, die Technetium sicher, fest und stabil in einer definierten Oxidationsstufe binden können, um zu verhindern, daß dadurch in vivo ablaufende Redoxprozesse bzw. Technetiumfreisetzungen aus dem entsprechenden Radiodiagnostika eine unerwünschte Biodistribution stattfindet, die eine sichere Diagnostik entsprechender Erkrankungen erschwert.

Als geeignete Komplexbildner für Technetium und Rheniumisotope gelten z. B. cyklische Amine (Troutner, D. E. et al.: J. Nucl. Med. 21, 443 (1980)), die aber den Nachteil haben, daß sie erst ab einem pH> 9 in der Lage sind Technetium-99m in guten Ausbeuten zu binden. $N_2O_2$-Systeme (Pillai, M. R. A., Troutner, D. E. et al.; Inorg. Chem. 29, 1850 (1990)) befinden sich in der klinischen Anwendung. Nichtcyklische $N_4$-Systeme wie z. B. das HMPAO haben als großen Nachteil ihre geringe Komplexstabilität. Tc-99m-HMPAO muß wegen seiner Instabilität (Ballinger, J. R. et al., Appl. Radiat. Isot. 42, 315 (1991); Billinghurst, M. W. et al., Appl. Radiat. Isot. 42, 607 (1991)) sofort nach seiner Markierung appliziert werden, damit der Anteil an Zerfallprodukten, die eine andere Pharmakokinetik und Ausscheidung besitzen, klein gehalten werden kann. Solche radiochemischen Verunreinigungen erschweren die Erkennung von zu diagnostizierenden Erkrankungen. Eine Kopplung dieser Chelate bzw. Chelatbildner an andere, sich selektiv in Krankheitsherden anreichernde Substanzen ist nicht mit einfachen Mitteln zu lösen, so daß sich diese im allgemeinen unspezifisch im Organismus verteilen.

$N_2S_2$-Chelatoren (Bormans, G. et al.; Nucl. Med. Biol., 17, 499 (1990)) wie z. B. Ethylendicystein (EC; Verbruggen, A. M. et al.; J. Nucl. Med. 33, 551 (1992)) erfüllen zwar die Forderung nach hinreichender Stabilität des entsprechenden Technetium-99m-Komplexes, bilden aber erst ab einem pH-Wert des Komplexierungsmediums >9 Radiodiagnostika mit einer Reinheit von größer 69 %. $N_3S$-Systeme (Fritzburg, A.; EPA 0 173 424 und EPA 0 250 013) bilden zwar stabile Technetium-99m-Komplexe, müssen aber zum Einbau des Radioisotops auf Temperaturen von ca. 100 °C erhitzt werden.

Ein weiterer Nachteil der $N_2S_2$- und $N_3S$-Systeme besteht darin, daß diese teilweise rasch und ohne spezifische Anreicherung vom Organismus ausgeschieden werden, so daß diese nur als Nierenfunktionsdiagnostika in der Klinik Anwendung finden und somit eine beschränkte Verwendbarkeit besitzen. In den letzten Jahren ist das Verlangen nach sich spezifisch in erkrankten Geweben anreichernden Radiodiagnostika gestiegen. Dies kann erreicht werden, wenn Komplexbildner leicht an sich selektiv anreichernden Substanzen gekoppelt werden können und dabei ihre günstigen Komplexierungseigenschaften nicht verlieren. Da es aber sehr häufig dazu kommt, daß nach Kopplung eines Komplexbildners unter Nutzung einer seiner funktionellen Gruppen an ein solches Molekül eine Abschwächung der Komplexstabilität beobachtet wird, erscheinen die bisherigen Ansätze zur Kupplung von Chelatbildnern an sich selektiv anreichernden Substanzen wenig zufriedenstellend, da ein diagnostisch nicht tolerierbarer Anteil des Isotops aus dem Konjugat in vivo freisetzt wird (Brechbiel, M. W. et al.; Inorg. Chem. 1986, 25, 2772). Es ist deswegen notwendig, bifunktionelle Komplexbildner darzustellen, die sowohl funktionelle Gruppen zur Bindung des gewünschten Metallions als auch eine (andere, mehrere) funktionelle Gruppe zur Bindung des sich selektiv anreichernden Moleküls tragen. Solche bifunktionellen Liganden ermöglichen eine spezifische, chemisch definierte Bindung von Technetium- oder Rhenium-Isotopen an verschiedenste biologische Materialien, auch dann, wenn ein sogenanntes Prelabeling durchgeführt wird. Es wurden einige Chelatbildner, gekoppelt an monoklonale Antikörper (z. B. EP Appl. 0 247 866 und EP Appl. 0 188 256) oder Fettsäuren (EP Appl. 0 200 492), beschrieben. Als Chelatbildner werden jedoch die bereits erwähnten $N_2S_2$-Systeme verwendet, die auf Grund ihrer geringen Stabilität wenig geeignet sind. Da sowohl die sich selektiv anreichernden Substanzen in ihren Eigenschaften, sowie auch die Mechanismen, nach denen sie angereichert werden, sehr unterschiedlich sind, ist es weiterhin notwendig, den kopplungsfähigen Chelatbildner zu variieren und den physiologischen Anforderungen des Kopplungspartners hinsichtlich seiner Lipo- und Hydrophilie, Membranpermeabilität bzw. -impermeabilität etc. anpassen zu können.

Der Erfindung lag daher die Aufgabe zugrunde, stabile Komplexverbindungen, die gekoppelt oder fähig zur Kopplung an unterschiedliche sich selektiv anreichernde Verbindungen sind, zur Verfügung zu stellen, sowie solche koppelbaren Chelatoren oder Komplexe bereitzustellen, die über eine größere chemische Variationsbreite der Substituenten verfügen, um diese den oben referierten Erfordernissen anpassen zu können. Des weiteren liegt der Erfindung die Aufgabe zugrunde, derartige Verbindungen und sie enthaltende pharmazeutische Mittel zur Verfügung zu stellen, sowie Verfahren zu ihrer Herstellung zu schaffen.

Überraschenderweise wird diese Aufgabe erfindungsgemäß dadurch gelöst, daß sich die neuen, ungewöhnlichen, bifunktionellen, chalkogenatom-unterbrochenen Chelatbildner und deren Kopplungsprodukte mit sich spezifisch anreichernden Verbindungen hervorragend zur Herstellung von Radiodiagnostika bzw. Radiotherapeutika eignen.

Gegenstand der Erfindung sind Verbindungen der allgemeinen Formel (I)

$$B\text{-}CO\text{-}CR^1R^2\text{-}A\text{-}CR^3R^4\text{-}CR^5R^6\text{-}A'\text{-}R^{12} \tag{I}$$

worin

A, A' gleich oder unterschiedlich sind und jeweils für ein Chalkogenatom O, S oder Se stehen,
$R^1$, $R^2$, $R^3$, $R^4$, $R^5$ und $R^6$ gleich oder unterschiedlich sind und jeweils für ein Wasserstoffatom und/oder für einen verzweigten oder unverzweigten $C_1$-$C_6$-Alkyrest stehen,
B einen Rest

$$\text{- NH-}(CR^7R^8)\text{-}(CR^9R^{10})_{n=1,2}\text{-S-}R^{11}$$

darstellt,
worin

$R^7$ und $R^8$ gleich oder unterschiedlich sind und jeweils ein Wasserstoffatom oder einen unverzweigten, verzweigten, cyclischen oder polycyclischen $C_1$-$C_{60}$-Alkyl-, Alkenyl-, Polyalkenyl-, Alkinyl-, Polyalkinyl-, Aryl-, Alkylaryl- oder Arylalkylrest darstellen, welcher gegebenenfalls mit Carboxy-, Aminocarbonyl-, Alkoxycarbonyl- ,Amino-, Aldehyd-, Hydroxy-, Oxo-, Oxy- oder Alkoxy-Gruppen mit bis zu 20 Kohlenstoffatomen substituiert ist und gegebenenfalls durch ein oder mehrere Heteroatome aus der Reihe O, N, S, P, As, Se unterbrochen und/oder substituiert ist,

$R^9$, $R^{10}$ gleich oder unterschiedlich sind und jeweils für ein Wasserstoffatom und/oder für einen verzweigten oder unverzweigten $C_1$-$C_6$-Alkylrest stehen,

$R^{11}$ für ein Wasserstoffatom, für eine Schwefelschutzgruppe, für Reste in der Bedeutung von $R^7$ oder $R^8$ steht

und $R^7$ und $R^{11}$ gegebenenfalls zusammen mit den sie verbindenden Gruppen einen gegebenenfalls mit Hydroxy-, Oxo-, Oxy- oder Alk-oxy-Gruppen mit bis zu 6 Kohlenstoffatomen substituierten 4-bis 8-gliedrigen Ring bilden,

$R^{12}$ ein Wasserstoffatom oder eine Chalkogenschutzgruppe ist, sowie deren Komplexe mit Radioisotopen von Tc oder Re.

Bevorzugte Verbindungen der allgemeinen Formel (I) zeichnen sich dadurch aus, daß A und A' Schwefelatome bedeuten und $R^{12}$ für ein Wasserstoffatom oder für eine Schwefelschutzgruppe steht.
Besonders bevorzugte erfindungsgemäße Verbindungen der allgemeinen Formel (I) zeichnen sich ferner dadurch aus, daß $R^7$ und $R^8$ unterschiedlich sind und $R^8$, $R^9$ und $R^{10}$ jeweils für ein Wasserstoffatom stehen.
Ein weiterer Gegenstand der Erfindung sind Konjugate, enthaltend eine Verbindung der allgemeinen Formel (I und sich selektiv in erkrankten Gewebe anreichernden Substanzen, wobei zwischen diesen eine kovalente Bindung besteht und diese im Falle von Carboxy- oder Aminogruppen enthaltenden Substanzen wie Peptiden, Proteinen, Antikörpern oder deren Fragmente amidisch oder im Falle von Hydroxygruppen enthaltenden Substanzen wie Fettalkoholen esterartig oder im Falle von Aldehydgruppen enthaltenden Substanzen imidisch vorliegt.
Besonders bevorzugte erfindungsgemäße Konjugate zeichnen sich dadurch aus, daß die sich in erkrankten Gewebe anreichernden Substanzen Peptide wie Endotheline, Teilsequenzen von Endothelinen, Endothelin-Analoga, Endothelin-Derivate oder Endothelin-Antagonisten bedeuten.
Bei weiteren bevorzugten Ausführungsformen der erfindungsgemäßen Konjugate weisen die Peptide die folgenden Sequenzen oder Teile davon

Cys-Ser-Cys-Ser-Ser-Leu-Met-Asp-Lys-Glu-Cys-Val-Tyr-
Phe-Cys-His-Leu-Asp-Ile-Ile-Trp,

Cys-Ser-Cys-Ser-Ser-Trp-Leu-Asp-Lys-Glu-Cys-Val-Tyr-
Phe-Cys-His-Leu-Asp-Ile-Ile-Trp,

Cys-Thr-Cys-Phe-Thr-Tyr-Lys-Asp-Lys-Glu-Cys-Val-Tyr-
Tyr-Cys-His-Leu-Asp-Ile-Ile-Trp,

Cys-Ser-Ala-Ser-Ser-Leu-Met-Asp-Lys-Glu-Ala-Val-Tyr-
Phe-Cys-His-Leu-Asp-Ile-Ile-Trp,

Cys-Ser-Cys-Asn-Ser-Trp-leu-Asp-Lys-Glu-Cys-Val-Tyr-
Phe-Cys-His-Leu-Asp-Ile-Ile-Trp,

Cys-Ser-Cys-Lys-Asp-Met-Thr-Asp-Lys-Glu-Cys-Leu-Asn-
Phe-Cys-His-Gln-Asp-Val-Ile-Trp

Ala-Ser-Cys-Ser-Ser-Leu-Met-Asp-Lys-Glu-Cys-Val-Tyr-
Phe-Ala-His-Leu-Asp-Ile-Ile-Trp,

Ala-Ser-Ala-Ser-Ser-Leu-Met-Asp-Lys-Glu-Ala-Val-Tyr-
Phe-Ala-His-Leu-Asp-Ile-Ile-Trp,

Cys-Ser-Cys-Ser-Ser-Trp-Leu-Asp-Lys-Glu-Ala-Val-Tyr-
Phe-Ala-His-Leu-Asp-Ile-Ile-Trp,

Cys-Val-Tyr-Phe-Cys-His-Leu-Asp-Ile-Ile-Trp,

N-Acetyl-Leu-Met-Asp-Lys-Glu-Ala-Val-Tyr-Phe-Ala-His-
Leu-Asp-Ile-Ile-Trp

die Teilsequenz

His-Leu-Asp-Ile-Ile-Trp

oder die cyclischen Aminosäuresequenzen

Cyclo-(DTrp-DAsp-Pro-DVal-Leu),

Cyclo-(DGlu-Ala-alloDIle-Leu-DTrp)

auf.

Die Herstellung der erfindungsgemäßen Verbindungen erfolgt dadurch, daß man Technetium-99m oder Re in Form von Pertechnetat oder Perrhenat in Gegenwart eines Reduktionsmittels und gegebenenfalls eines Hilfsliganden mit Verbindung der allgemeinen Formel (I)

$$B\text{-}CO\text{-}CR^1R^2\text{-}A\text{-}CR^3R^4\text{-}CR^5R^6\text{-}A'\text{-}R^{12} \tag{I}$$

worin $R^1$, $R^2$, $R^3$, $R^4$, $R^5$, $R^6$, A, A' und B die vorstehend angegebene Bedeutung haben, umsetzt.

Die Herstellung der erfindungsgemäßen Liganden der allgemeinen Formel (I) erfolgt dadurch, daß man Verbindungen der allgemeinen Formel (II)., mit Verbindungen der allgemeinen Formel gemäß nachfolgendem Reaktionsschema

$$X\text{-}CO\text{-}CR^1R^2\text{-}A\text{-}CR^3R^4\text{-}CR^5R^6\text{-}A'\text{-}R^{12} \tag{II}$$

$$+ NH_2\text{-}CR^7R^8\text{-}(CR^9R^{10})_{n=1,2}\text{-}S\text{-}R^{11} \tag{III}$$

$$\rightarrow B\text{-}CO\text{-}CR^1R^2\text{-}A\text{-}CR^3R^4\text{-}CR^5R^6\text{-}A'\text{-}R^{12} \tag{I}$$

umsetzt,
worin

X für eine Abgangsgruppe steht und $R^1$, $R^2$, $R^3$, $R^4$, $R^5$, $R^6$, $R^7$, $R^8$, $R^9$, $R^{10}$, $R^{11}$, A, A' und B die in vorstehend angegebene Bedeutung haben.

Diese Umsetzungen werden in polaren und unpolaren, aprotischen Lösungsmitteln wie beispielsweise Dichlormethan, Tetrahydrofuran, Chloroform, 1,4-Dioxan, DMF, oder DMSO bei Temperaturen zwischen - 30 und + 100 °C unter Zugabe einer Hilfsbase zum Abfangen der freiwerdenden Säuren durchgeführt. Solche können beispielsweise sein: tertiäre Amine, Alkali- und Erdalkalihydroxide, Alkali- und Erdalkalicarbonate.

Ein weiterer Gegenstand der Erfindung ist ein Kit, zur Herstellung von Radiopharmaka, bestehend aus einer Verbindung der allgemeinen Formel (I) oder einem erfindungsgemäßen Konjugat enthaltend Verbindungen der allgemeinen Formel (I) und sich selektiv in Geweben anreichernden Substanzen einem Reduktionsmittel und gegebenenfalls einem Hilfsliganden, die in trockenem Zustand oder in Lösung vorliegen, einer Gebrauchsanweisung mit einer Reaktionsvorschrift zur Umsetzung der beschriebenen Verbindungen mit Technetium-99m oder Re in Form einer Pertechnetatlösung oder Perrhenatlösung.

Gegenstand der Erfindung ist auch eine radiopharmazeutische Zusammensetzung zur nicht invasiven in vivo Darstellung von Rezeptoren und rezeptorhaltigem Gewebe und/oder von atherosklerotischen Plaques. Sie enthält eine Verbindung der allgemeinen Formel (I) oder ein erfindungsgemäßes Konjugat enthaltend Verbindungen der allgemeinen Formel (I) und sich selektiv In Geweben anreichernden Substanzen, gegebenenfalls mit den in der Galenik üblichen Zusätzen,wobei die Verbindung in einem Kit mit Technetium-99m oder Re in Form einer Pertechnetatlösung oder Perrhenatlösung zubereitet wird.

Ein weiterer Gegenstand der Erfindung ist eine Methode zur Durchführung einer radiodiagnostischen Untersuchung, wobei die radiopharmazeutische Zusammensetzung in einer Menge von 0,1 bis 30 mCi, bevorzugt von 0,5 bis 10 mCi pro 70 kg Körpergewicht einem Patienten verabreicht und die vom Patienten abgegebene Strahlung aufgezeichnet wird.

Überraschenderweise zeigten viele der synthetisierten und mit Tc-99m oder Re markierten Chelate eine höhere Stabilität als vergleichbare $N_2S_2$ und $N_3S$-Systeme, die in der Literatur beschrieben sind. So konnten z. B. bei einer erfindungsgemäßen Substanz (Beispiel 3 a, 3 b), die an ein Fettalkohol gekoppelt wurde, keine Zersetzungsprodukte nach 26 h beobachtet werden. Auch konnte durch Kompetitionsversuche festgestellt werden, daß die in dieser Erfindung beschriebenen Tc-99m oder Re Chelatoren besser als die vergleichbaren $N_2S_2$, $N_3S$ und Propylenaminoxinum-Systeme komplexieren. Die in der vorliegenden Erfindung beschriebenen Chelate und Chelatbildner sind damit eindeutig besser für diagnostische und therapeutische Zwecke geeignet als die bisher bekannten Systeme. Ein besonderer Vorteil der erfindungsgemäßen Chelatoren besteht darin, daß deren Synthesen ohne Verwendung von Schwefelschutzgruppen geführt werden können. Dies macht deren Synthese sehr einfach und zusätzlich bieten speziell solche erfindungsgemäß beschriebenen Verbindungen den Vorteil, daß nach radiochemischer Markierung keine weiteren Fremdmoleküle in den zur Radiodiagnostik bzw. Radiotherapie, z. B. intravenös zu applizierenden Lösungen, enthalten sind, die häufig die Biodistribution des Radipharmakons stören und damit den diagnostischen Informationsgehalt nachteilig beeinflussen können. Außerdem können die Markierungen an solchen Liganden bzw. deren Kopplungsprodukte an sich selektiv in erkrankten Geweben anreichernden Substanzen unter sehr milden Bedingungen vorgenommen werden. So gelingt die Markierung der erfindungsgemäßen Liganden bzw. der Kopplungsprodukte an sich selektiv in erkrankten Geweben anreichernden Substanzen bei Raumtemperatur und bei physiologischem pH-Wert, ohne daß vorher unter Einwirkung von Basen, Säuren oder anderen dem Fachmann bekannten Hilfsstoffen, die Schutzgruppen abzuspalten wären. Dies bietet Gewähr, daß durch solche Hilfsstoffe die häufig sehr empfindlichen, sich selektiv in erkrankten Geweben anreichernden Substanzen nicht chemisch verändert werden, was häufig deren selektive Anreicherung in erkranktem Gewebe herabsetzt und somit den Informationsgehalt bei der Radiodiagnostik nachteilig beeinflussen würde.

Dennoch können natürlich auch hier Schwefelschutzgruppen Verwendung finden, wenn die eben geschilderten Nachteile in Kauf genommen werden können. Deren Etablierung an Schwefelatomen bzw. deren Abspaltung geschieht dann nach Methoden, die dem Fachmann bekannt sind. Die Kopplung an sich selektiv in erkrankten Geweben anreichernden Substanzen erfolgt ebenfalls nach an sich dem Fachmann bekannten Methoden (z. B. Fritzberg et al.; J. Nucl. Med. $\underline{26}$, 7 (1987)), beispielsweise durch Reaktion von elektrophilen Gruppen des Komplexliganden mit nukleophilen Zentren der sich selektiv in erkrankten Geweben anreichernden Substanzen. Ansonsten werden nukleophile Gruppen des Chelators mit elektrophilen Gruppen der sich selektiv in erkrankten Geweben anreichernden Substanzen gekoppelt.

Als Kopplungspartner sind u. a. verschiedene Biomoleküle vorgesehen, Liganden, die an spezifische Rezeptoren binden und so ein in ihrer Rezeptordichte verändertes Gewebe erkennen können, hierzu gehören u. a. Peptide und Steroidhormone, Wachstumsfaktoren, Neurotransmitter. Mit Liganden für Steroidhormonrezeptoren wurde die Möglichkeit einer verbesserten Diagnostik von Brust- und Prostatakarzinomen aufgezeigt (S. J. Brandes and J. A. Katzenellenbogen, Nucl. Med. Biol. $\underline{15}$, 53, 1988).

Verschiedentlich weisen Tumorzellen eine veränderte Dichte von Rezeptoren für Peptidhormone oder Wachstumsfaktoren, wie z. B. den "epidermal growth factor" (EgF) auf. Die Konzentrationsunterschiede konnten zur selektiven Anreicherung von Cytostatika in Tumorzellen genutzt werden (E. Aboud-Pirak et al., Proc. Natl. Acad. Sci., USA 86; 3778,1989). Vielfach konnten mit Positronen-emittierenden Isotopen markierte Liganden für Neurorezeptoren zur Diagnostik verschiedener Hirnerkrankungen herangezogen werden (J. J. Forst, Trends in Pharmacol. Sci., $\underline{7}$, 490, 1989). Weitere Biomoleküle sind in den Metabolismus der Zellen einschleusbare Metabolite, die einen veränderten Stoffwechsel erkennbar machen; hierzu gehören beispielsweise Fettsäuren, Saccharide, Peptide und Aminosäuren. Fettsäuren gekoppelt an die instabileren $N_2S_2$-Chelatbildner wurden in der EPA 0 200 492 beschrieben. Andere Stoffwechselprodukte wie Saccharide (Desoxyglucose), Lactat, Pyruvat und Aminosäuren (Leucin, Methylmethionin, Glycin) wurden mit Hilfe der PET-Technik zur bildlichen Darstellung von veränderten Stoffwechselvorgängen herangezogen (R. Weinreich, Swiss Med., $\underline{8}$, 10, 1986). Auch nicht biologische Substanzen wie Misonidazol und seine Derivate, die sich in Geweben bzw. Gewebeteilen mit reduzierter Sauerstoffkonzentration irreversibel an Zellbestandteilen binden, können zur spezifischen Anreicherung von radioaktiven Isotopen und somit zur bildlichen Darstellung von Tumoren oder ischämischen Regionen herangezogen werden (M. E. Shelton, J. Nucl. Med. $\underline{30}$; 351, 1989). Schließlich ist auch die Kopplung der bifunktionellen Chelatbildner an monoklonale Antikörper bzw. derer Fragmente möglich. Besonders günstig erwiesen sich Kopplungsprodukte der erfindungsgemäßen Chelatoren bzw. deren Komplexe mit Technetium -99m oder Re mit Fettalkoholen, Fettalkoholderivaten oder mit Fettaminen bzw. deren Derivate, oder mit Endothelinen, Teilsequenzen von Endothelinen, Endothelin-Analoga, Endothelin-Derivaten oder Endothelin-Antagonisten zur Detektion von atherosklerotischen Gefäßerkrankungen. Die Derivate wurden WHHL-Kaninchen appliziert, die durch einen genetischen Defekt des LDL-Rezeptors hohe LDL-Konzentrationen im Blut aufweisen und somit atherosklerotische Läsionen aufweisen. Etwa 4 bis 5 h nach i. V. Applikation der Derivate in WHHL-Kaninchen konnten Anreicherungsquotienten im Vergleich zu nicht geschädigtem Gewebe von 3 bis 40 in den atheromatösen Plaques nachgewiesen werden. Dadurch können atherosklerotische Gefäßbereiche mit den in der Radiodiagnostik üblichen Methoden (z. B. Gamma-Szintillationskamera) nachgewiesen werden. Bisher konnten nur sehr späte Stadien der Atherogenese mit invasiveren Verfahren (z. B. Arteriographie) diagnostiziert werden. Die erfindungsgemäßen Substanzen bieten deshalb den entscheidenden Vorteil viel frühere Stadien der Atherosklerose mit nicht invasiveren Verfahren zu diagnostizieren.

Es ist unerheblich, ob eine Markierung der Chelatbildner mit Tc-99m oder Rhenium-Isotopen vor oder nach der Kopplung an das sich selektiv anreichernde Molekül durchgeführt wird. Für eine Kopplung an das sich selektiv anreichernde Molekül nach einer Komplexierung ist jedoch Voraussetzung, daß die Umsetzung des radioaktiven Komplexes mit der sich anreichernden Verbindung schnell, unter schonenden Bedingungen und nahezu quantitativ abläuft, so daß keine anschließende Aufreinigung erforderlich ist.

Die Herstellung der erfindungsgemäßen pharmazeutischen Mittel erfolgt in an sich bekannter Weise, in dem man die erfindungsgemäßen Komplexbildner unter Zusatz eines Reduktionsmittels, vorzugsweise Zinn(II)-salzen wie - chlorid oder -tartrat - und gegebenenfalls unter Zugabe der in der Galenik üblichen Zusätze - in wäßrigem Medium löst und anschließend sterilfiltriert. Geeignete Zusätze sind beispielsweise physiologisch unbedenkliche Puffer (z. U. Tromethamin), geringe Zusätze von Elektrolyten (z. B. Natriumchlorid), Stabilisatoren (z. B. Gluconat, Phosphate oder Phosphonate). Das erfindungsgemäße pharmazeutische Mittel liegt in Form einer Lösung oder in lyophilisierter Form vor und wird kurz vor der Applikation mit einer Lösung Tc-99m-Pertechnetat, eluiert aus kommerziell erhältlichen Generatoren, oder einer Perrhenatlösung versetzt.

Bei der nuklearmedizinischen in-vivo-Anwendung werden die erfindungsgemäßen Mittel in Mengen von $1.10^{-5}$ bis $5.10^{4}$ nmol/kg Körpergewicht, vorzugsweise in Mengen zwischen $1.10^{-3}$ bis $5.10^{2}$ nmol/kg Körpergewicht dosiert. Ausgehend von einem mittleren Körpergewicht von 70 kg beträgt die Radioaktivitätsmenge für diagnostische Anwendungen zwischen 0,05 und 50 mCi, vorzugsweise 5 bis 30 mCi pro Applikation. Für therapeutische Anwendungen werden zwischen 5 und 500 mCi, vorzugsweise 10 - 350 mCi appliziert. Die Applikation erfolgt normalerweise durch intravenöse, intraarterielle, peritoneale oder intratumorale Injektion von 0,1 bis 2 ml einer Lösung der erfindungsgemäßen Mittel. Bevorzugt ist die intravenöse Applikation.

Die nachfolgenden Beispiele dienen der näheren Erläuterung des Erfindungsgegenstandes.

**Beispiel 1 a**

**2,5-Dithia-cyclohexanon**

Zu einer gerührten Lösung aus 51 ml (0,6 mol) 1,2 -Dimercaptoethan und 168 ml (1,2 mol) Triethylamin in 600 ml wasserfreiem Dichlormethan tropft man unter Eiskühlung 48 ml (0,6 mol) Chloracetylchlorid, gelöst in 300 ml wasserfreiem Dichlormethan. Anschließend rührt man 3 h bei Raumtemperatur. Das Triethylaminhydrochlorid wird abgefiltert und die organische Phase mit Wasser gewaschen. Nach Trocknung über Magnesiumsulfat wird das Lösungsmittel unter vermindertem Druck verdampft und der Rückstand im Vakuum destilliert. (In Anlehnung an J. Larsen et. al. Synthesis 1989, 134).

Ausbeute: 52 g (65 %), gelbliches Öl

| Analyse: | | | | |
|---|---|---|---|---|
| Ber.: | C 35,80 | H 4,51 | O 11,92 | S 47,76 |
| Gef.: | C 35,63 | H 4,68 | | S 47,49 |

**Beispiel 1 b**

**S-(2-Mercaptoethyl)-N-(2-mercapto-1-(methoxy-carbonyl)-ethyl)-mercaptoessigsäureamid**

Zu einer Lösung aus 17,16 g (0,1 mol) Cysteinmethylesterhydrochlorid und 10,12 g (0,1 mol) Triethylamin in 500 ml wasserfreiem Dichlormethan unter Argonatmosphäre tropft man 13,42 g (0,1 mol) 2,5-Dithia-cyclohexanon, gelöst in 250 ml wasserfreiem Dichlormethan. Man läßt das Reaktionsgemisch über Nacht bei Raumtemperatur rühren, wäscht jeweils dreimal mit 2-%iger wäßriger Citronensäure, gesättigter Natriumhydrogencarbonatlösung und mit Wasser. Nach Trocknung über Natriumsulfat wird das Lösungsmittel unter vermindertem Druck verdampft. Der ölige Rückstand wird durch Verreiben mit Diethylether kristallisiert.

Ausbeute: 21,32 g (79,1 %), weißes Pulver

| Analyse: | | | | | |
|---|---|---|---|---|---|
| Ber.: | C 35,67 | H 5,61 | N 5,20 | O 17,82 | S 35,70 |

(fortgesetzt)

| Analyse: | | | | | |
|---|---|---|---|---|---|
| Gef.: | C 35,48 | H 5,72 | N 4,97 | | S 35,43 |

**Beispiel 1 c**

**S-(2-Mercaptoethyl)-N-(2-mercapto-1-(methoxy-carbonyl)-ethyl)-mercaptoessigsäureamid, Technetium-99m-Komplex**

10 mg des unter Beispiel 1 b hergestellten Liganden werden in 1,0 ml 0,5 M Phosphatpuffer pH 7,5 gelöst. 50 µl dieser Ligand-Lösung werden mit 250 µl PhosphatPuffer pH 7,5, 50 µl einer desoxygenierten wäßrigen Citratlösung (50 mg/ml), 2.5 µl einer desoxygenierten wäßrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist >98 %.

**Beispiel 2 a**

**S-(2-Mercaptoethyl)-N-(2-mercapto-1-(hydroxycarbonyl)-ethyl)-mercaptoessigsäureamid**

2,69 g (10 mmol) des unter Beispiel 1 b hergestellten Liganden werden in 200 ml 2N wäßriger Natriumhydroxidlösung unter Argonatmosphäre gelöst. Nach 2 h Rühren bei Raumtemperatur stellt man mit Argon gesättigter, konzentrierter Salzsäure pH = 3 ein und extrahiert das ausgeschiedene Öl erschöpfend mit Essigester. Nach Trocknung über Natriumsulfat wird das Lösungsmittel unter vermindertem Druck verdampft und der ölige Rückstand wird durch Verreiben mit Diethylether zur Kristallisation gebracht.

Ausbeute: 732 mg (28,7 %), weißes Pulver

| Analyse: bezogen auf die wasserfreie Substanz | | | | | |
|---|---|---|---|---|---|
| Ber.: | C 32,92 | H 5,13 | N 5,49 | O 18,80 | S 37,66 |
| Gef.: | C 32,73 | H 5,38 | N 5,30 | | S 37,41 |

**Beispiel 2 b**

**S-(2-Mercaptoethyl)-N-(2-mercapto-1-(hydroxycarbonyl)-ethyl)-mercaptoessigsäureamid, Technetium-99m-Komplex**

10 mg des unter Beispiel 2 a hergestellten Liganden werden in 1,0 ml 0,5 M Phosphat-Puffer pH 7,5 gelöst. 50 µl dieser Ligand-Lösung werden mit 250 µl PhosphatPuffer pH 7,5, 50 µl einer desoxygenierten wäßrigen Citratlösung (50 mg/ml), 2,5 µl einer desoxygenierten wäßrigen Zinn(II)chlorid-Lösung (5 mg/ml 0,05 N HCl) und 100 µl einer Pertechnetatlösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist>98 %.

**Beispiel 3 a**

**S-(2-Mercaptoethyl)-N-(2-mercapto-1-(decyloxycarbonyl)-ethyl)-mercaptoessigsäureamid**

Zu einer Lösung aus 29,79 g (0,1 mol) Cysteindecylesterhydrochlorid und 10,12 g (0,1 mol) Triethylamin in 500 ml wasserfreiem Dichlormethan unter Argonatmosphäre tropft man 13,42 g (0,1 mol) 2,5-Dithia-cyclohexanone, gelöst in 250 ml wasserfreiem Dichlormethan. Man läßt das Reaktionsgemisch über Nacht bei Raumtemperatur rühren, wäscht jeweils dreimal mit 2-%iger wäßriger Citronensäure, gesättigter Natriumhydrogencarbonatlösung und mit Wasser. Nach Trocknung über Natriumsulfat wird das Lösungsmittel unter vermindertem Druck verdampft. Der ölige Rückstand wird nach Verreiben mit Diethylether kristallisiert.

Ausbeute: 31,45 g (79,5 %), weißes Pulver

| Analyse: | | | | | |
|---|---|---|---|---|---|
| Ber.: | C 51,61 | H 8,41 | N 3,54 | O 12,13 | S 24,31 |
| Gef.: | C 51,48 | H 8,52 | N 3,40 | | S 24,05 |

**Beispiel 3 b**

**S-(2-Mercaptoethyl)-N-(2-mercapto-1-(decyloxycarbonyl)-ethyl)-mercaptoessigsäureamid, Technetium-99m-Komplex**

10 mg des unter Beispiel 3 a hergestellten Liganden werden in 1,0 ml Ethanol gelöst, 50 μl dieser Ligand-Lösung werden mit 250 μl Phosphat-Puffer pH 8,5, 50 μl einer desoxygenierten wäßrigen Citratlösung (50 mg/ml), 2, 5 μl einer desoxygenierten wäßrigen Zinn(II)chlorid-Lösung (5 mg/ml 0,05 N HCl) und 100 μl einer Pertechnetat-Lösung (400 - 900 μCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 μm, 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist >95 %.

**Beispiel 4 a**

**S-(2-Mercaptoethyl)-N-(2-mercapto-1-(2-methoxyethoxycarbonyl)-ethyl)-mercaptoessigsäureamid**

2,69 g (10 mmol) des unter Beispiel 1 b beschriebenen Liganden werden in Gegenwart von 190 mg (1 mmol) Toluolsulfonsäurehydrat unter Argonatmosphäre in 250 ml wasserfreiem Ethylenglycolmonomethylether 6 h unter Rückfluß erhitzt. Anschließend verdampft man das Lösungsmittel unter vermindertem Druck und nimmt den öligen Rückstand in Dichlormethan auf. Die Dichlormethanlösung wird jeweils dreimal mit 2-%iger wäßriger Citronensäure, gesättigter Natriumhydrogencarbonatlösung und mit Wasser gewaschen. Nach Trocknung über Natriumsulfat verdampft man das Lösungsmittel unter vermindertem Druck und chromatographiert das Öl an Kieselgel (Eluens: Dichlormethan/Methanol 8 : 2). Abschließend wird aus Diethylether kristallisiert.

Ausbeute: 632 mg (22,2 %), weißes Pulver

| Analyse: | | | | | |
|---|---|---|---|---|---|
| Ber.: | C 38,32 | H 6,11 | N 4,47 | O 20,42 | S 30,68 |
| Gef.: | C 38,14 | H 6,37 | N 4,18 | | S 30,41 |

**Beispiel 4 b**

**S-(2-Mercaptoethyl)-N-(2-mercapto-1-(2-methoxyethoxycarbonyl)-ethyl)-mercaptoessigsäureamid, Technetium-99m-Komplex**

10 mg des unter Beispiel 4 a hergestellten Liganden werden in 1,0 ml Ethanol gelöst. 50 μl dieser Ligand-Lösung werden mit 250 μl Phosphat-Puffer pH 8,5, 50 μl einer desoxygenierten wäßrigen Citratlösung (50 mg/ml), 2,5 µl einer desoxygenierten wäßrigen Zinn(II)chlorid-Lösung (5 mg/ml 0,05 N HCl) und 100 µl einer Pertechnetatlösung (400 - 900 µCi) versetzt. Das Reaktionsgemisch wird nach einer Inkubationszeit von 10 min mittels HPLC auf die Reinheit des gebildeten Tc-Komplexes untersucht. Hamilton PRP-1 Säule, 5 µm, 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist >95 %.

**Beispiel 5 a**

**S-(2-Mercaptoethyl)-N-(2-mercapto-1-(octylaminocarbonyl)-ethyl)-mercaptoessigsäureamid**

Zu einer Lösung aus 2,69 g (10 mmol) des unter Beispiel 1 b hergestellten Liganden in 30 ml Ethanol gibt man 70 ml n-Octylamin und erhitzt das Reaktionsgemisch 6 h unter Argonatmosphäre zum Sieden. Anschließend wird im Feinvakuum eingedampft, der Rückstand mit 200 ml 2-%iger wäßriger Citronensäure und 200 ml Dichlormethan unter Argonatmosphäre versetzt. Die Mischung wird 15 min heftig verrührt, die Dichlormethanphase abgetrennt und jeweils dreimal mit 2-%iger wäßriger Citronensäure, gesättigter Natriumhydrogencarbonatlösung und mit Wasser gewaschen. Nach Trocknung über Natriumsulfat verdampft man das Lösungsmittel unter vermindertem Druck und chromatographiert den Rückstand an Kieselgel (Eluens: Dichlormethan/Methanol 95 : 5). Abschließend wird aus Diethylether umkristallisiert.

Ausbeute: 548 mg (14,9 %), weißes Pulver

| Analyse: | | | | | |
|---|---|---|---|---|---|
| Ber.: | C 49,15 | H 8,25 | N 7,64 | O 8,73 | S 26,24 |
| Gef.: | C 49,00 | H 8,31 | N 7,66 | | S 26.02 |

**Beispiel 5 b**

**S-(2-Mercaptoethyl)-N-(2-mercapto-1-(octylaminocarbonyl)-ethyl)-mercaptoessigsäureamid, Technetium-99m-Komplex**

10 mg des unter Beispiel 5 a hergestellten Liganden werden in 1,0 ml Ethanol gelöst. 50 µl dieser Ligand-Lösung werden mit 250 µl Phosphat-Puffer pH 8,5, 50 µl einer desoxygenierten wäßrigen Citratlösung (50 mg/ml), 2,5 µl einer desoxygenierten wäßrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradientenelution von 100 % A nach 100 % B innerhalb von 7,5 min (Eluent A: Natriumhydrogenphosphat 0,005 M, pH 7,4: Eluent B: Acetonitril/Natriumhydrogenphosphat 0,005 M, pH 7,4 (75/25); 2,0 ml/min. Die radiochemische Reinheit ist > 95 %.

**Beispiel 6 a**

**S-(2-Mercaptoethyl)-N-(2-mercapto-1-(2,3 dihydroxypropylaminocarbonyl)-ethyl)-mercaptoessigsäureamid**

2,69 g (10 mmol) des unter Beispiel 1 b hergestellten Liganden werden in 30 ml Ethanol und 30 ml Aminopropandiol 7 h unter Argonatmosphäre zum Sieden erhitzt. Anschließend wird unter vermindertem Druck das Ethanol abdestilliert, der Rückstand mit argongesättigtem Wasser versetzt und anschließend mit argongesättigter konzentrierter Salzsäure ein pH von 7 eingestellt. Die gelbliche Lösung wird gefriergetrocknet und der Rückstand an Kieselgel RP 18 (Eluens: Wasser, Tetrahydrofuran, Tetrahyperofuran 0 - 50 %) chromatographiert. Nach Verdampfen des Lösungsmittels erhält man ein farbloses Glas.

Ausbeute: 378 mg (10 %), farbloses Glas

| Analyse: bezogen auf die wasserfreie Substanz | | | | | |
|---|---|---|---|---|---|
| Ber.: | C 36,57 | H 6,14 | N 5,53 | O 19,48 | S 29,28 |
| Gef.: | C 36,31 | H 6,47 | N 8,34 | | S 29,01 |

**Beispiel 6 b**

**S-(2-Mercaptoethyl)-N-(2-mercapto-1-(2,3 dihydroxypropylaminocarbonyl)-ethyl)-mercaptoessigsäureamid, Technetium-99m-Komplex**

10 mg des unter Beispiel 6 a hergestellten Liganden werden in 1,0 ml Ethanol gelöst. 50 µl dieser Ligand-Lösung werden mit 250 µl Phosphat-Puffer pH 8,5, 50 µl einer desoxygenierten wäßrigen Citratlösung (50 mg/ml), 2,5 µl einer desoxygenierten wäßrigen Zinn(II)-chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm. Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist >95 %.

**Beispiel 7 a**

**S-(2-Mercapto-ethyl)-N-(2-mercapto-1-(carbonyl-His-Leu-Asp-Ile-Ile-Trp)-ethyl)-mercaptoessigsäureamid**

Zu einer Lösung von 900 mg (1 mmol) $NH_2$-Cys-His-Leu-Asp-Ile-Ile-Trp (hergestellt in Analogie zu Barany und Merrifield, The Peptides: Analysis, Biology, Academic Press, New York, 1980; Stewart und Young, Solid Phase Peptides Syntheses, 2[nd]ed., Pierce Chemical W., Rockford, Il, 1984) und 304 mg (3 mmol) Triethylamin in 100 ml wasserfreiem Dimethylformamid unter Argonatmosphäre gibt man 134 mg (1 mmol) 2,5-Dithia-cyclohexanon (Beispiel 1 a) und rührt das resultierende Reaktionsgemisch 13 h bei Raumtemperatur. Nach beendeter Reaktion wird filtriert und das Lösungsmittel unter vermindertem Druck abgezogen. Das verbleibende Öl wird dreimal mit 50 ml Dimethylformamid versetzt und jeweils eingedampft. Man verrührt den Rückstand in 100 ml wasserfreiem Diethylether, worauf sich ein weißer Feststoff abscheidet, der abfiltriert wird. Zur Reinigung wird aus Dimethylformamid/Diethylethergemischen umkristallisiert.

Ausbeute: 423 mg (40,9 %), weißes Pulver

| Analyse: bezogen auf die wasserfreie Substanz | | | | | |
|---|---|---|---|---|---|
| Ber.: | C 53,47 | H 6,63 | N 13,56 | O 17,03 | S 9,31 |
| Gef.: | C 53,19 | H 6,92 | N 13,28 | | S 8,97 |

**Beispiel 7 b**

**S-(2-Mercapto-ethyl)-N-(2-mercapto-1-(carbonyl-His-Leu-Asp-Ile-Ile-Trp)-ethyl-mercaptoessigsäureamid, Technetium-99m-Komplex**

10 mg des unter Beispiel 7 a hergestellten Liganden werden in 1,0 ml Ethanol gelöst. 50 µl dieser Ligand-Lösung werden mit 250 µl Phosphat-Puffer pH 8,5 50 µl einer desoxygenierten wässrigen Citratlösung (50 mg/ml), 2,5 µl einer desoxygenierten wässrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradientenelution von 100 % A nach 100 % B innerhalb von 7,5 min (Eluent A: Natriumhydrogenphosphat, 0,005 M, pH 7,4; Eluent B: Acetonitril/Natriumhydrogenphosphat 0,005 M, pH 7,4 (75/25); 2,0 ml/min. Die radiochemische Reinheit ist > 95 %.

**Beispiel 8**

**Anreicherung des S-(2-Mercapto-ethyl)-N-(2-mercapto-1-(decyloxycarbonyl)-ethyl)-mercaptoessigsäureamids, Technetium-99m-Komplex in atherosklerotischen Gefäßläsionen von WHHL-Kaninchen**

Die Markierung des S-(2-Mercapto-ethyl)-N-(2-mercapto-1-(decyloxycarbonyl)-ethyl)-mercaptoessigsäureamids (hergestellt nach Beispiel 3 a) erfolgt wie in Beispiel 3 b beschrieben.

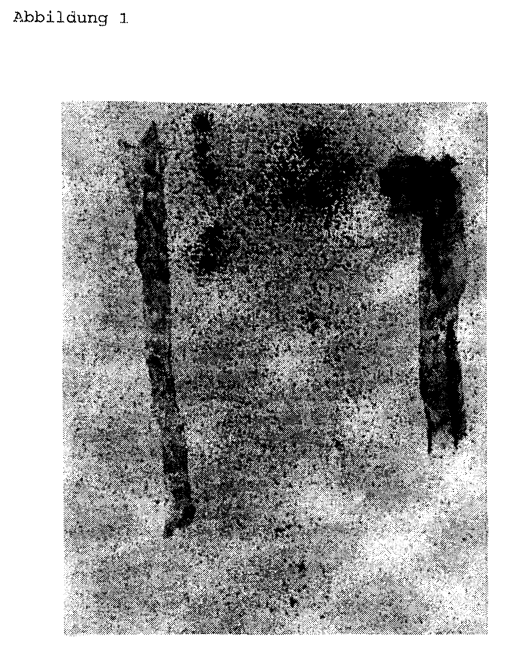

99,9 GBq (2,7 mCi) der nach Beispiel 3 b markierten Substanz wurde mit phosphatgepufferter Saline auf 1 ml verdünnt und einem narkotisierten WHHL-Kaninchen Rompun/Ketavet (1:2) über eine Ohrvene appliziert. 5 h nach Applikation wurde das Kaninchen getötet und sowohl eine Autoradiographie der Aorta als auch eine Sudan-III-Färbung zur Darstellung der atheroskıerotischen Plaques durchgeführt (Abbildung 1). Der Anreicherungsfaktor zwischen normalen und atherosklerotischen Wandbereichen betrug je nach Ausbildung der Plaques (Sudan-III-Färbung) zwischen 3 und 8.

**Patentansprüche**

1.   Verbindungen der allgemeinen Formel (I)

$$B\text{-}CO\text{-}CR^1R^2\text{-}A\text{-}CR^3R^4\text{-}CR^5R^6\text{-}A'\text{-}R^{12} \hspace{3cm} (I)$$

worin

A, A' gleich oder unterschiedlich sind und jeweils für ein Chalkogenatom O, S oder Se stehen,
$R^1$, $R^2$, $R^3$, $R^4$, $R^5$ und $R^6$ gleich oder unterschiedlich sind und jeweils für ein Wasserstoffatom und/oder für einen verzweigten oder unverzweigten $C_1$-$C_6$-Alkylrest stehen,
B einen Rest

$$-NH\text{-}(CR^7R^8)\text{-}(CR^9R^{10})_{n=1,2}\text{-}S\text{-}R^{11,}$$

darstellt,
worin
$R^7$ und $R^8$ gleich oder unterschiedlich sind und jeweils ein Wasserstoffatom oder einen unverzweigten, verzweigten, cyclischen oder polycyclischen $C_1$-$C_{60}$-Alkyl-, Alkenyl-, Polyalkenyl-, Alkinyl-, Polyalkinyl-, Aryl-, Alkylaryl- oder Arylalkyl-Rest darstellen, welcher gegebenenfalls mit Hydroxy-, Oxy-, Oxo-, Carboxy-, Aminocarbonyl-, Alkoxycarbonyl-, Amino-, Aldehyd- oder Alkoxy-Gruppen mit bis zu 20 Kohlenstoffatomen substituiert ist und/oder gegebenenfalls durch ein oder mehrere Heteroatome aus der Reihe O, N, S, P, As, Se unterbrochen und/oder substituiert ist,
$R^9$ und $R^{10}$ gleich oder unterschiedlich sind und jeweils für ein Wasserstoffatom und/oder für einen verzweigten oder unverzweigten $C_1$-$C_6$-Alkylrest stehen,
$R^{11}$ für ein Wasserstoffatom, für eine Schwefelschutzgruppe, für Reste in der Bedeutung von $R^7$ oder $R^8$ steht oder $R^7$ und $R^{11}$ zusammen mit den sie verbindenden Gruppen einen gegebenenfalls mit Hydroxy-, Oxo-, Oxy- oder Alkoxy-Gruppen mit bis zu 6 Kohlenstoffatomen substituierten 4- bis 8-gliedrigen Ring bilden,
$R^{12}$ ein Wasserstoffatom oder eine Chalkogenschutzgruppe ist

deren Konjugate mit sich selektiv in erkranktem Gewebe anreichernde Substanzen, wobei zwischen diesen eine kovalente Bindung besteht und diese im Falle von Carboxy- oder Aminogruppen enthaltenden Substanzen wie Peptiden, Proteinen und Antikörpern oder deren Fragmenten, amidisch oder im Falle von Hydroxygruppen enthaltenden Substanzen wie Fettalkoholen, esterartig oder im Falle von Aldehydgruppen enthaltenden Substanzen imidisch vorliegt
sowie deren Komplexe mit Radioisotopen von Tc oder Re.

2.   Verbindungen nach Anspruch 1, dadurch gekennzeichnet, daß A und A' Schwefelatome bedeuten und $R^{12}$ für ein Wasserstoffatom oder für eine Schwefelschutzgruppe steht.

3.   Verbindungen nach Anspruch 1 oder 2, dadurch gekennzeichnet, daß $R^7$ und $R^8$ unterschiedlich sind und $R^8$, $R^9$ und $R^{10}$ jeweils für ein Wasserstoffatom stehen.

4.   Verbindungen nach mindestens einem der Ansprüche 1 bis 3, dadurch gekennzeichnet, daß die sich in erkranktem Gewebe anreichernden Substanzen Peptide wie Endotheline, Teilsequenzen von Endothelinen, Endothelin-Analoga, Endothelin-Derivate oder Endothelin-Antagonisten bedeuten.

5. Verbindungen nach mindestens einem der Ansprüche 1 bis 4, dadurch gekennzeichnet, daß die Peptide die folgenden Sequenzen oder Teile davon aufweisen

Cys-Ser-Cys-Ser-Ser-Leu-Met-Asp-Lys-Glu-Cys-Val-Tyr-

Phe-Cys-His-Leu-Asp-Ile-Ile-Trp,

Cys-Ser-Cys-Ser-Ser-Trp-Leu-Asp-Lys-Glu-Cys-Val-Tyr-

Phe-Cys-His-Leu-Asp-Ile-Ile-Trp,

Cys-Thr-Cys-Phe-Thr-Tyr-Lys-Asp-Lys-Glu-Cys-Val-Tyr-

Tyr-Cys-His-Leu-Asp-Ile-Ile-Trp,

Cys-Ser-Ala-Ser-Ser-Leu-Met-Asp-Lys-Glu-Ala-Val-Tyr-

Phe-Cys-His-Leu-Asp-Ile-Ile-Trp,

```
   ┌────────────────────────────────────────────┐
Cys-Ser-Cys-Asn-Ser-Trp-Leu-Asp-Lys-Glu-Cys-Val-Tyr-
   └───┐
Phe-Cys-His-Leu-Asp-Ile-Ile-Trp,
```

```
                ┌────────────────────────────────────────────┐
Cys-Ser-Cys-Lys-Asp-Met-Thr-Asp-Lys-Glu-Cys-Leu-Asn-
   └───┐
Phe-Cys-His-Gln-Asp-Val-Ile-Trp,
```

```
                ┌────────────────────────────────────────────┐
Ala-Ser-Cys-Ser-Ser-Leu-Met-Asp-Lys-Glu-Cys-Val-Tyr-
Phe-Ala-His-Leu-Asp-Ile-Ile-Trp,
```

```
Ala-Ser-Ala-Ser-Ser-Leu-Met-Asp-Lys-Glu-Ala-Val-Tyr-
Phe-Ala-His-Leu-Asp-Ile-Ile-Trp,
```

```
Cys-Ser-Cys-Ser-Ser-Trp-Leu-Asp-Lys-Glu-Ala-Val-Tyr-
Phe-Ala-His-Leu-Asp-Ile-Ile-Trp,
```

```
   ┌──────────────────────┐
Cys-Val-Tyr-Phe-Cys-His-Leu-Asp-Ile-Ile-Trp,
```

```
N-Acetyl-Leu-Met-Asp-Lys-Glu-Ala-Val-Tyr-Phe-Ala-
His-Leu-Asp-Ile-Ile-Trp,
```

die Teilsequenz

His-Leu-Asp-Ile-Ile-Trp

oder die cyclischen Aminosäuresequenzen

Cyclo-(DTrp-DAsp-Pro-DVal-Leu),
Cyclo-(DGlu-Ala-alloDIle-Leu-DTrp)

aufweisen.

6. Verfahren zur Herstellung der Verbindungen nach Anspruch 1, dadurch gekennzeichnet, daß man Verbindungen der allgemeinen Formel (II) mit Verbindungen der allgemeinen Formel (III) gemäß nachfolgendem Reaktionsschema

$$X\text{-}CO\text{-}CR^1R^2\text{-}A\text{-}CR^3R^4\text{-}CR^5R^6\text{-}A'\text{-}R^{12} \qquad\qquad (II)$$

$$+NH_2-CR^7R^8-(CR^9CR^{10})_{n=1,2}-S-R^{11} \qquad \text{(III)}$$

$$\rightarrow B-CO-CR^1R^2-A-CR^3R^4-CR^5R^6-A'-R^{12} \qquad \text{(I)}$$

umsetzt,
worin

X für eine Abgangsgruppe steht und $R^1$, $R^2$, $R^3$, $R^4$, $R^5$, $R^6$, $R^7$, $R^8$, $R^9$, $R^{10}$, $R^{11}$, $R^{12}$, A, A' und B die im Anspruch 1 angegebene Bedeutung haben
und daß

gegebenenfalls die so hergestellten Verbindungen mit sich selektiv in erkranktem Gewebe oder in Tumoren anreichernden Substanzen konjugiert werden, wobei zwischen diesen eine kovalente Bindung geknüpft wird, welche im Falle von Aminogruppen enthaltenden Substanzen wie Peptiden, Proteinen und Antikörpern, amidisch oder im Falle von Hydroxygruppen enthaltenden Substanzen wie Alkoholen, esterartig oder im Falle von Aldehydgruppen enthaltenden Substanzen imidisch erfolgt,
und daß

die so hergestellten Verbindungen und Konjugate mit Technetium-99m oder Re in Form von Pertechnetat oder Perrhenat in Gegenwart eines Reduktionsmittels und gegebenenfalls eines Hilfsliganden umgesetzt werden.

7.  Radiopharmazeutische Zusammensetzung zur nicht invasiven in-vivo-Darstellung von Rezeptoren und rezeptorhaltigem Gewebe und/oder von atherosklerotischen Plaques, dadurch gekennzeichnet, daß sie eine Verbindung oder ein Konjugat gemäß einem der Ansprüche 1 bis 5, sowie gegebenenfalls mit den in der Galenik üblichen Zusätzen, enthält.

**Claims**

1.  Compounds of the general formula (I)

$$B-CO-CR^1R^2-A-CR^3R^4-CR^5R^6-A'-R^{12} \qquad \text{(I)},$$

wherein

A and A' are the same or different and each represents a chalcogen atom O, S or Se, $R^1$, $R^2$, $R^3$, $R^4$, $R^5$ and $R^6$ are the same or different and each represents a hydrogen atom and/or a branched or unbranched $C_1$-$C_6$alkyl radical,

B represents a radical

$$-NH-(CR^7R^8)-(CR^9R^{10})_{n=1,2}-S-R^{11},$$

wherein

$R^7$ and $R^8$ are the same or different and each represents a hydrogen atom or an unbranched or branched, cyclic or polycyclic $C_1$-$C_{60}$-alkyl, -alkenyl, -polyalkenyl, -alkynyl, -polyalkynyl, -aryl, -alkylaryl or -arylalkyl radical, which is optionally substituted by hydroxy, oxy, oxo, carboxy, aminocarbonyl, alkoxycarbonyl, amino, aldehyde or alkoxy group(s) having up to 20 carbon atoms and/or optionally interrupted and/or substituted by one or more hetero atoms from the series O, N, S, P, As and Se,

$R^9$ and $R^{10}$ are the same or different and each represents a hydrogen atom and/or a branched or unbranched $C_1$-$C_6$alkyl radical,

$R^{11}$ represents a hydrogen atom, a sulphur-protecting group, or one of the radicals given for $R^7$ or $R^8$, or

$R^7$ and $R^{11}$ together with the groups connecting them form a 4- to 8-membered ring optionally substituted by hydroxy, oxo, oxy or alkoxy group(s) having up to 6 carbon atoms, and

$R^{12}$ is a hydrogen atom or a chalcogen-protecting group,

conjugates thereof with substances that accumulate selectively in diseased tissue, wherein the compounds and those substances are covalently bonded, the covalent bond being an amide bond in the case of substances containing carboxy or amino groups, such as peptides, proteins and antibodies or fragments thereof, or an ester-type bond in the case of substances containing hydroxy groups, such as fatty alcohols, or an imide bond in the case of substances containing aldehyde groups,

and complexes thereof with radioisotopes of Tc or Re.

2. Compounds according to claim 1, characterised in that A and A' represent sulphur atoms and $R^{12}$ denotes a hydrogen atom or a sulphur-protecting group.

3. Compounds according to claim 1 or 2, characterised in that $R^7$ and $R^8$ are different and $R^8$, $R^9$ and $R^{10}$ each represent a hydrogen atom.

4. Compounds according to at least one of claims 1 to 3, characterised in that the substances that accumulate in diseased tissue are peptides, such as endothelins, endothelin partial sequences, endothelin analogues, endothelin derivatives or endothelin antagonists.

5. Compounds according to at least one of claims 1 to 4, characterised in that the peptides have the following sequences or portions thereof

```
        ┌────────────────────────────────┐
Cys-Ser-Cys-Ser-Ser-Leu-Met-Asp-Lys-Glu-Cys-Val-Tyr-
        └───┐
Phe-Cys-His-Leu-Asp-Ile-Ile-Trp,
```

Cys-Ser-Cys-Ser-Ser-Trp-Leu-Asp-Lys-Glu-Cys-Val-Tyr-

Phe-Cys-His-Leu-Asp-Ile-Ile-Trp,


Cys-Thr-Cys-Phe-Thr-Tyr-Lys-Asp-Lys-Glu-Cys-Val-Tyr-

Tyr-Cys-His-Leu-Asp-Ile-Ile-Trp,


Cys-Ser-Ala-Ser-Ser-Leu-Met-Asp-Lys-Glu-Ala-Val-Tyr-

Phe-Cys-His-Leu-Asp-Ile-Ile-Trp,


Cys-Ser-Cys-Asn-Ser-Trp-Leu-Asp-Lys-Glu-Cys-Val-Tyr-

Phe-Cys-His-Leu-Asp-Ile-Ile-Trp,


Cys-Ser-Cys-Lys-Asp-Met-Thr-Asp-Lys-Glu-Cys-Leu-Asn-

Phe-Cys-His-Gln-Asp-Val-Ile-Trp,


Ala-Ser-Cys-Ser-Ser-Leu-Met-Asp-Lys-Glu-Cys-Val-Tyr-
Phe-Ala-His-Leu-Asp-Ile-Ile-Trp,


Ala-Ser-Ala-Ser-Ser-Leu-Met-Asp-Lys-Glu-Ala-Val-Tyr-
Phe-Ala-His-Leu-Asp-Ile-Ile-Trp,


Cys-Ser-Cys-Ser-Ser-Trp-Leu-Asp-Lys-Glu-Ala-Val-Tyr-
Phe-Ala-His-Leu-Asp-Ile-Ile-Trp,


Cys-Val-Tyr-Phe-Cys-His-Leu-Asp-Ile-Ile-Trp,

N-acetyl-Leu-Met-Asp-Lys-Glu-Ala-Val-Tyr-Phe-Ala-
His-Leu-Asp-Ile-Ile-Trp,

the partial sequence

His-Leu-Asp-Ile-Ile-Trp

or the cyclic amino acid sequences

cyclo-(DTrp-DAsp-Pro-DVal-Leu),

cyclo- (DGlu-Ala-alloDIle-Leu-DTrp).

6. Process for the preparation of the compounds according to claim 1, characterised in that compounds of the general formula (II) are reacted with compounds of the general formula (III) in accordance with the following reaction scheme

$$X\text{-}CO\text{-}CR^1R^2\text{-}A\text{-}CR^3R^4\text{-}CR^5R^6\text{-}A'\text{-}R^{12} \tag{II}$$

$$+ NH_2\text{-}CR^7R^8\text{-}(CR^9R^{10})_{n=1,2}\text{-}S\text{-}R^{11} \tag{III}$$

$$\rightarrow B\text{-}CO\text{-}CR^1R^2\text{-}A\text{-}CR^3R^4\text{-}CR^5R^6\text{-}A'\text{-}R^{12} \tag{I},$$

wherein X is a leaving group and $R^1$, $R^2$, $R^3$, $R^4$, $R^5$, $R^6$, $R^7$, $R^8$, $R^9$, $R^{10}$, $R^{11}$, $R^{12}$, A, A' and B have the meanings given in claim 1,
and

optionally the compounds so prepared are conjugated with substances that accumulate selectively in diseased tissue or in tumours, wherein the compounds and those substances are covalently bonded, the covalent bond being an amide bond in the case of substances containing amino groups, such as peptides, proteins and antibodies, or an ester-type bond in the case of substances containing hydroxy groups, such as alcohols, or an imide bond in the case of substances containing aldehyde groups
and

the compounds and conjugates so prepared are reacted with technetium-99m or Re in the form of pertechnetate or perrhenate in the presence of a reducing agent and, optionally, an auxiliary ligand.

7. Radiopharmaceutical composition for non-invasive *in vivo* visualisation of receptors and receptor-containing tissue and/or of atherosclerotic plaques, characterised in that it comprises a compound or a conjugate according to any one of claims 1 to 5, optionally together with the additives customary in galenical pharmacy.

**Revendications**

1. Composés de la formule générale (I)

$$B\text{-}CO\text{-}CR^1R^2\text{-}A\text{-}CR^3R^4\text{-}CR^5R^6\text{-}A'\text{-}R^{12} \tag{I}$$

dans laquelle

A, A' sont identiques ou différents et représentent chacun un atome de chalcogène O, S ou Se,
$R^1$, $R^2$, $R^3$, $R^4$, $R^5$ et $R^6$ sont identiques ou différents et représentent chacun un atome d'hydrogène et/ou un radical alkyle en $C_1$-$C_6$ ramifié ou non ramifié,
B représente un radical

$$-NH-(CR^7R^8)-(CR^9R^{10})_{n=1,2}-S-R^{11}$$

où

$R^7$ et $R^8$ sont identiques ou différents et représentent chacun un radical arylalkyle, alkylaryle, aryle, polyalcynyle, alcynyle, polyalcényle, alcényle, alkyle en $C_1$-$C_{60}$ cyclique ou polycyclique, ramifié ou non ramifié, qui est éventuellement substitué par des groupes hydroxy, oxy, oxo, carboxy, aminocarbonyle, alcoxycarbonyle, amino, aldéhyde ou alcoxy comportant jusqu'à 20 atomes de carbone et/ou est éventuellement interrompu et/ou substitué par un ou plusieurs hétéroatomes de la série O, N, S, P, As, Se,

$R^9$ et $R^{10}$ sont identiques ou différents et représentent chacun un atome d'hydrogène et/ou un radical alkyle en $C_1$-$C_6$ ramifié ou non ramifié, $R^{11}$ représente un atome d'hydrogène, un groupe de protection de soufre, des radicaux de la signification de $R^7$ ou $R^8$, ou $R^7$ et $R^{11}$ forment conjointement avec les groupes qui les relient un noyau rectangulaire à octogonal éventuellement substitué par des groupes hydroxy, oxo, oxy ou alcoxy comportant jusqu'à 6 atomes de carbone,

$R^{12}$ représente un atome d'hydrogène ou un groupe de protection de chalcogène,

leurs produits de conjugaison avec des substances qui s'enrichissent de manière sélective dans un tissu atteint de maladie, une liaison covalente existant entre ces derniers et celle-ci étant de type amide, dans le cas de substances contenant des groupes carboxy ou amino, comme des peptides, des protéines et des anticorps ou leurs fragments, ou de type ester, dans le cas de substances contenant des groupes hydroxy, comme des alcools gras, ou de type imide, dans le cas de substances contenant des groupes aldéhyde,

ainsi que leurs complexes avec des radioisotopes de Tc ou de Re.

2. Composés suivant la revendication 1, caractérisé en ce que A et A' représentent des atomes de soufre et $R^{12}$ représente un atome d'hydrogène ou un groupe de protection de soufre.

3. Composés suivant l'une des revendications 1 et 2, caractérisés en ce que $R^7$ et $R^8$ sont différents et en ce que $R^8$, $R^9$ et $R^{10}$ représentent chacun un atome d'hydrogène.

4. Composés suivant au moins l'une des revendications 1 à 3, caractérisé en ce que les substances qui s'enrichissent dans un tissu atteint de maladie représentent des peptides, tels que des endothélines, des séquences partielles d'endothélines, des analogues d'endothélines, des dérivés d'endothélines ou des antagonistes d'endothélines.

5. Composés suivant au moins l'une des revendications 1 à 4, caractérisés en ce que les peptides présentent les séquences suivantes ou des parties de celles-ci

Cys-Ser-Cys-Ser-Ser-Leu-Met-Asp-Lys-Glu-Cys-Val-Tyr-
Phe-Cys-His-Leu-Asp-Ile-Ile-Trp,

Cys-Ser-Cys-Ser-Ser-Trp-Leu-Asp-Lys-Glu-Cys-Val-Tyr-
Phe-Cys-His-Leu-Asp-Ile-Ile-Trp,

Cys-Thr-Cys-Phe-Thr-Tyr-Lys-Asp-Lys-Glu-Cys-Val-Tyr-
Tyr-Cys-His-Leu-Asp-Ile-Ile-Trp,

Cys-Ser-Ala-Ser-Ser-Leu-Met-Asp-Lys-Glu-Ala-Val-Tyr-
Phe-Cys-His-Leu-Asp-Ile-Ile-Trp,

Cys-Ser-Cys-Asn-Ser-Trp-Leu-Asp-Lys-Glu-Cys-Val-Tyr-
Phe-Cys-His-Leu-Asp-Ile-Ile-Trp,

Cys-Ser-Cys-Lys-Asp-Met-Thr-Asp-Lys-Glu-Cys-Leu-Asn-
Phe-Cys-His-Gln-Asp-Val-Ile-Trp,

Ala-Ser-Cys-Ser-Ser-Leu-Met-Asp-Lys-Glu-Cys-Val-Tyr-
Phe-Ala-His-Leu-Asp-Ile-Ile-Trp,

Ala-Ser-Ala-Ser-Ser-Leu-Met-Asp-Lys-Glu-Ala-Val-Tyr-
Phe-Ala-His-Leu-Asp-Ile-Ile-Trp,

```
Cys-Ser-Cys-Ser-Ser-Trp-Leu-Asp-Lys-Glu-Ala-Val-Tyr-
Phe-Ala-His-Leu-Asp-Ile-Ile-Trp,
```

```
┌──────────────────────┐
Cys-Val-Tyr-Phe-Cys-His-Leu-Asp-Ile-Ile-Trp,
```

```
N-Acetyl-Leu-Met-Asp-Lys-Glu-Ala-Val-Tyr-Phe-Ala-
His-Leu-Asp-Ile-Ile-Trp,
```

la séquence partielle

His-Leu-Asp-Ile-Ile-Trp

ou les séquences d'acides aminés cycliques

Cyclo-(DTrp-DAsp-Pro-DVal-Leu),
Cyclo-(DGlu-Ala-alloDIle-Leu-DTrp)

6. Procédé de préparation des composés suivant la revendication 1, caractérisé en ce qu'on fait réagir des composés de la formule générale (II) avec des composés de la formule générale (III) conformément au schéma réactionnel suivant

$$X\text{-}CO\text{-}CR^1R^2\text{-}A\text{-}CR^3R^4\text{-}CR^5R^6\text{-}A'\text{-}R^{12} \qquad (II)$$

$$+NH_2\text{-}CR^7R^8\text{-}(CR^9CR^{10})_{n=1,2}\text{-}S\text{-}R^{11} \qquad (III)$$

$$\rightarrow B\text{-}CO\text{-}CR^1R^2\text{-}A\text{-}CR^3R^4\text{-}CR^5R^6\text{-}A'\text{-}R^{12} \qquad (I)$$

où

X représente un groupe de départ et $R^1$, $R^2$, $R^3$, $R^4$, $R^5$, $R^6$, $R^7$, $R^8$, $R^9$, $R^{10}$, $R^{11}$, $R^{12}$, A, A' et B ont la signification indiquée dans la revendication 1, et en ce qu'éventuellement on conjugue les composés ainsi préparés avec des substances qui s'enrichissent de manière sélective dans un tissu atteint de maladie ou dans des tumeurs, une liaison covalente étant nouée entre ceux-ci, liaison qui a lieu à la manière d'un amide dans le cas de substances contenant des groupes amino, comme des peptides, des protéines et des anticorps, ou à la manière d'un ester, dans le cas de substances contenant des groupes hydroxy, comme des alcools, ou à la manière d'un imide, dans le cas de substances contenant des groupes aldéhyde, et en ce que les composés et produits de conjugaison ainsi préparés sont amenés à réagir avec du technétium-99m ou du Re sous la forme de pertechnétate ou de perrhénate en présence d'un réducteur et éventuellement d'un ligand auxiliaire.

7. Composition radiopharmaceutique pour la représentation in vivo non invasive de récepteurs et de tissus contenant des récepteurs et/ou de plaques de type athérosclérose, caractérisée en ce qu'elle contient un composé ou un produit de conjugaison suivant l'une des revendications 1 à 5, ainsi qu'éventuellement les additifs courants en pharmacie galénique.

Abbildung 1